Europäisches Patentamt

European Patent Office

Office européen des brevets

(19)

(11) Veröffentlichungsnummer: **0 226 107 B1**

(12) **EUROPÄISCHE PATENTSCHRIFT**

(45) Veröffentlichungstag der Patentschrift: 07.08.91

(51) Int. Cl.⁵: **A61F 7/00**, F25D 3/10, F17C 9/02

(21) Anmeldenummer: 86116633.8

(22) Anmeldetag: 29.11.86

(54) **Verfahren zur Erzeugung einer Behandlungsatmosphäre für die Behandlung rheumatisch erkrankter Personen durch Tieftemperatur und zur Durchführung des Verfahrens eine Anlage für die Ganzkörperbehandlung.**

(30) Priorität: 03.10.86 DE 3633637
07.12.85 DE 3543390

(43) Veröffentlichungstag der Anmeldung:
24.06.87 Patentblatt 87/26

(45) Bekanntmachung des Hinweises auf die Patenterteilung:
07.08.91 Patentblatt 91/32

(84) Benannte Vertragsstaaten:
AT BE CH DE ES FR GB GR IT LI NL SE

(56) Entgegenhaltungen:
EP-A- 0 192 971      CA-A- 1 143 581
DE-B- 3 242 881      GB-A- 2 005 000
US-A- 2 943 459      US-A- 3 714 942
US-A- 3 807 396      US-A- 4 165 618
US-A- 4 344 291

(73) Patentinhaber: **Nusec Anlagenbau GmbH**
**Uffelnsweg 22**
**W-2000 Hamburg 28(DE)**

(72) Erfinder: **Blaudszun,Bernd,Dipl.Ing.**
**Burgerei 57a**
**W-2162 Steinkirchen(DE)**
Erfinder: **Dittmar, Bernd**
**Im Wortchen 2**
**W-3105 Fassberg/Muden(DE)**

(74) Vertreter: **Dipl.-Ing. H. Hauck, Dipl.-Ing. E. Graalfs, Dipl.-Ing. W. Wehnert, Dr.-Ing. W. Döring**
**Neuer Wall 41**
**W-2000 Hamburg 36(DE)**

## Beschreibung

Die Erfindung bezieht sich auf ein Verfahren zur Erzeugung einer Behandlungsatmosphäre für die Behandlung rheumatisch erkrankter Personen durch Tieftemperaturtherapie nach dem Oberbegriff des Patentanspruchs 1.

Aus der DE-OS 32 42 881 oder der DE-PS 33 05 434 ist bekanntgeworden, rheumatisch erkrankte Glieder mit tiefkaltem Gas zu behandeln. Aus der DE-OS 32 13 919, welche den gleichen Gegenstand betrifft, ist bekannt, trockene Luft mit Flüssigstickstoff im Wärmeaustausch zu kühlen. Dieses Verfahren ist jedoch verhältnismäßig aufwendig. Bei den zuvor genannten Verfahren wird Trägergas, vorwiegend Luft, mit Hilfe von schlagartig verdampftem flüssigem Stickstoff heruntergekühlt, bevor das dann kalte Gas gegen einen Körperteil gerichtet wird. Aus der DE-OS 32 42 881 ist auch bekannt, das erzeugte kalte Gas in eine Behandlungskabine zu leiten, in der sich ein Patient aufhalten kann. Zu diesem Zweck enthält das Trägergas zum Beispiel 50% Sauerstoff und 50% Stickstoff, während das Flüssiggas aus flüssigem Stickstoff besteht. Bei der Mischung beider Gase wird dann ein kaltes Gasgemisch erhalten, das einen der Luft entsprechenden Sauerstoffgehalt aufweist. Das zuletzt genannte Verfahren ist mit einigen Nachteilen verbunden.

Trägergas mit einer Mischung 50% Sauerstoff und 50% Stickstoff ist nicht marktgängig, muß daher speziell hergestellt werden. Bei der Herstellung einer Atmosphäre für eine Behandlungskabine sind dabei sowohl das Mischungsverhältnis von Sauerstoff und Stickstoff im Trägergas als auch die Sauerstoffkonzentration im Kaltgas zu überwachen. Ist indessen bei einer Abweichung von der vorgegebenen Konzentration die Zufuhr an Trägergas zu erhöhen, wird dadurch gleichzeitig die Temperatur des kalten Gases beeinflußt, die jedoch ihrerseits einen vorgegebenen Wert aufweisen soll. Es sind daher Vorkehrungen zu treffen, das Ansteigen der Temperatur bei einer Zunahme an Trägergas durch entsprechende Kühlung zu kompensieren. Bei dem bekannten Verfahren wird die Temperatur durch die Menge an Flüssiggas bestimmt. Steigt indessen die Temperatur in der Behandlungskabine aufgrund der erhöhten Zufuhr an Trägergas an, kann nicht gleichzeitig mehr Flüssiggas zugeführt werden, weil dadurch wieder die gewünschte Konzentration beeinflußt wird. Umgekehrt kann bei dem bekannten Verfahren nicht ohne weiteres eine Temperaturregelung durchgeführt werden, da eine Änderung der Zufuhr an Flüssiggas auch die Zusammensetzung der Behandlungsatmosphäre verändert.

Der Erfindung liegt die Aufgabe zugrunde, ein Verfahren zur Erzeugung einer Behandlungsatmosphäre für die Behandlung rheumatisch erkrankter Personen durch Tieftemperaturtherapie anzugeben, das zu seiner Durchführung nur einen minimalen Aufwand erfordert und eine einfache Regelung der Konzentration und der Temperatur ermöglicht.

Diese Aufgabe wird durch die Merkmale des Kennzeichnungsteils des Patentanspruchs 1 gelöst.

Bei erfindungsgemäßen Verfahren werden von vornherein flüssiger Sauerstoff und flüssiger Stickstoff verwendet, die ohne weiteres im Handel erhältlich sind. Vorrichtungen zur Trocknung der Atmosphäre oder Kältemaschinen zur Herstellung der Behandlungsatmosphäre können in Fortfall kommen. Flüssiger Stickstoff und flüssiger Sauerstoff werden entweder in der flüssigen oder in der gasförmigen Phase gemischt, wobei entweder nach dem Mischen oder vor dem Mischen eine Verdampfung stattfindet. Diese läßt sich jedoch relativ einfach durchführen, beispielsweise durch Wärmeaustausch mit Umgebungstemperatur. Durch Volumenstromregelung läßt sich die gewünschte Konzentration von Sauerstoff in der Behandlungsatmosphäre präzise einstellen bzw. regeln. Die Regelung ist dabei unabhängig von der Temperatur, die dadurch eingestellt werden kann, daß die Erwärmung des kalten Behandlungsgases gesteuert wird.

Von weiterem Vorteil ist, daß die Behandlungsatmosphäre einen tiefen Taupunkt besitzt und es dadurch erst relativ spät zu Vereisungen in der Behandlungskabine kommt. Die Zeit bis zum Start des erfindungsgemäßen Verfahrens, d.h. bis zum Behandlungsbeginn ist relativ gering.

Nach einer Ausgestaltung der Erfindung kann eine Taupunktregelung auch dadurch erfolgen, daß die Abluftmenge aus der Behandlungskabine geregelt wird.

Vor einer Therapie kann die Behandlungskabine dadurch auf einen gewünschten Taupunkt gebracht werden, daß die Behandlungskabine mit der Behandlungsatmosphäre bei Normaltemperatur beschickt bzw. gespült wird. Da die Behandlungsatmosphäre naturgemäß sehr trocken ist, wird dadurch relativ rasch in der Behandlungskabine vorhandene Feuchtigkeit entfernt.

Vorteilhafte Ausgestaltungen des erfindungsgemäßen Verfahrens sind in weiteren Unteransprüchen angegeben.

Die Anlage zur Durchführung des erfindungsgemäßen Verfahrens geht von einer Behandlungskabine aus, der eine Mischvorrichtung für kalte Gase vorgeschaltet ist. Die erfindungsgemäße Anlage sieht mindestens zwei Lagertanks für flüssigen Stickstoff und flüssigen Sauerstoff vor, die jeweils mittels einer Leitung mit Volumenstromreglern mit einer Mischkammer verbunden sind, deren Ausgang mit mindestens einem Lagertank für die Behandlungsatmosphäre verbunden ist, der seinerseits über eine geregelte Nachheizeinrichtung mit der Behandlungskabine verbunden ist.

Alle mit tiefen Temperaturen arbeitenden Anlagekomponenten sind in einer weiteren Ausgestaltung der Erfindung vakuumisoliert. Insbesondere kann die Behandlungskabine von Doppelwänden umgeben sein, wobei trockenes Tieftemperaturgas in den Zwischenraum eingeleitet wird.

Weitere vorteilhafte Ausgestaltungen der erfindungsgemäßen Anlage sind in weiteren Unteransprüchen angegeben.

Die Erfindung wird anhand von Zeichnungen näher erläutert.
Es zeigt:

Fig. 1   das Schaltbild einer Anlage für eine Ganzkörpertherapie,

Fig. 2   einen Wärmetauscher für eine exakte Temperaturregelung

Fig. 3   die Ausbildung des Wärmetauscherrohrs eines Wärmetauschers für die Vorrichtung nach Fig. 2,

Fig. 4   den Temperaturverlauf an einem Wärmetauscherrohr nach Fig. 3,

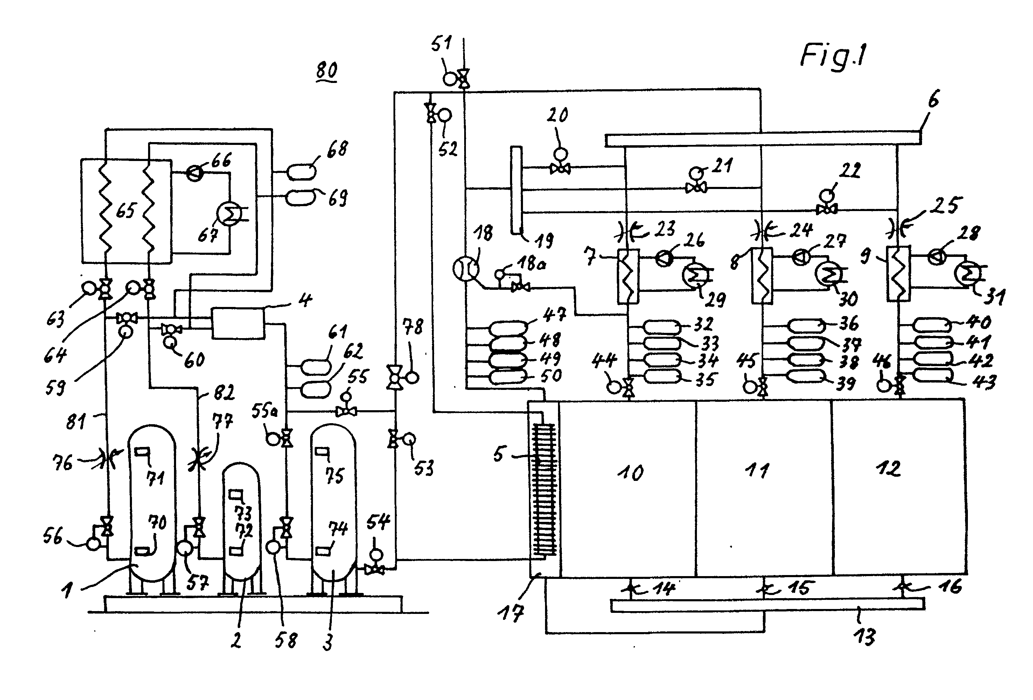

Das Verfahren für eine Ganzkörpertherapie wird nachstehend anhand der in Fig. 1 schematisch dargestellten Anlage 80 erläutert. Die zur Herstellung der Behandlungsatmosphäre benötigten Gase Stickstoff und Sauerstoff werden in den Lagertanks 1, 2 bevorratet. Die Lagertemperatur dieser Gase ist für Sauerstoff - 183 °C und für Stickstoff - 196 °C. Die Lagertanks 1, 2 sind vorzugsweise vakuumisoliert, um die Verdampfungsverluste so gering wie möglich zu halten. Die Konstantdruckregulierung beider Lagertanks 1, 2 erfolgt über die Regler 56, 57, damit eine exakte Vermischung beider Gase erreicht wird. Der Volumenstrom der Sauerstoff- und Stickstoffatmosphären wird mittels der Volumenstromregler 76, 77 geregelt. Es sind zwei Mischvarianten möglich, einmal die Mischung von Stickstoff und Sauerstoff in der flüssigen Phase und zum anderen das Mischen der Behandlungsatmosphäre in der Gasphase.

Bei der Mischung von Stickstoff und Sauerstoff in der flüssigen Phase sind die Absperrventile 63, 64 geschlossen, der Kreislaufventilator 66 und der Verdampfer 65 außer Betrieb und das Heizregister 67 für den Verdampfer 65 ausgeschaltet. Die Absperrventile 59, 60 sind geöffnet und der flüssige Sauerstoff und Stickstoff durchströmt die Mischkammer 4 mit konstantem geregelten Druck und Volumenstrom. Nach Austritt aus der Mischkammer 4 wird durch Meßgeräte 61, 62 die Sauerstoffkonzentration und der Taupunkt des flüssigen Gemisches analysiert und nach Bedarf mittels der Volumenstromregler 76, 77 korrigiert, so daß der Sollwert erreicht wird.

Der Lagertank 3 wird mit der gewünschten Behandlungsatmosphäre befüllt, wobei das Absperrventil 55 geschlossen und das Absperrventil 55a geöffnet ist. Nach Erreichen des Füllniveaus, das von einer Mindestniveaukontrolle 74 bzw. einer Maximumniveaukontrolle 75 angezeigt wird, werden die Absperrventile 59, 60 vor dem Eintritt der Mischkammer 4 geschlossen. Nunmehr ist im Lagertank 3 die ausreichende Menge an gewünschter Behandlungsatmosphäre vorhanden.

Beim Start der Anlage 80 ist folgendes zu beachten. Bevor die Klimakammern 10, 11, 12, auf die gewünschte Temperatur abgesenkt werden, muß deren gesamte Atmosphäre mit trockener Behandlungsatmosphäre solange durchspült werden, bis sich der gewünschte Taupunkt in den Klimakammern eingestellt hat. Dies ist erforderlich um eine Vereisung der Klimakammern zu verhindern. Hierbei sind die Absperrventile 54, 53 zu öffnen und das Absperrventil 52 zu schließen. Die noch flüssige Behandlungsatmosphäre durchströmt den Sammler 6 und wird gleichmäßig auf die Klimakammern 10, 11, 12 verteilt. Es ist möglich, jede gewünschte Anzahl von Klimakammern zu betreiben, so daß auch mehr als drei Klimakammern zum Einsatz kommen können.

Die Temperaturregelkammern 7, 8, 9 der Klimakammern 10, 11, 12 werden mit voller Leistung der Kreislaufgebläse 26, 27, 28 betrieben, wobei die Heizregister 29, 30, 31 ihre maximale Leistung erreichen. Die Volumenstromregler 23, 24, 25 für die Volumenstromregelung der Behandlungsatmosphäre sind voll geöffnet. Die Absperrventile 44, 45, 46 sind ebenfalls offen. Der Gasstrahlventilator 18 wird über den Treibstrahlregler 18a aktiviert und saugt aus den Klimakammern 10, 11, 12 die eintretende Atmosphäre ab. Hierbei muß das Absperrventil 51 geöffnet sein. Die Regelklappen 14, 15, 16 für die Abluft der Klimakammern 10, 11, 12 sind voll geöffnet. Nach erreichen des gewünschten Taupunktes, was von den Meßgeräten 33, 37, 41 analysiert wird, sind die Klimakammern 10, 11, 12 betriebsbereit und können kalt gefahren werden. Hierzu wird das Absperrventil 53 geschlossen. Die flüssige Behandlungsatmosphäre aus dem Lagertank 3 durchströmt den Vorverdampfer 5 und gelangt über das geöffnete Absperrventil 52 in den Sammler 6. In den als Wärmetauscher ausgebildeten Temperaturregelkammern 7, 8, 9 wird die gewünschte Temperatur für die einzelnen Klimakammern 10, 11, 12 über die Meßgeräte 35, 39, 43 von Temperaturreglern geregelt. Die Absperrventile 44, 45, 46 sind offen. Die Regelklappen 14, 15, 16 für die Abluft sind weiterhin voll geöffnet. Dies gilt auch für das Absperrventil 51. Die Regelventile 20, 21, 22 für die den Klimakammern 10, 11, 12 zuzuführende Rückluft sind geschlossen. Der Gasstrahlventilator 18 ist weiterhin in Betrieb. Nach erreichen der Solltemperaturen in den einzelnen Klimakammern 10, 11, 12 ist die Anlage 80 für die Therapie freigegeben.

Während des Betriebs der Anlage 80 werden permanent die wichtigen Parameter der Behandlungsatmosphäre wie Temperatur über Meßgeräte 35, 39, 43, 50, Sauerstoffkonzentration über Meßgeräte 32, 36, 40, 47, $CO_2$-Konzentration über Meßgeräte 34, 38, 42, 49 und auch der Taupunkt über Meßgeräte 33, 37, 41, 48 überwacht und geregelt. Die Abluft aus dem Sammler 13 wird vor Rückführung in die Klimakammern durch Meßgeräte 47, 48, 49, 50 analysiert. Je nach dem, welcher Abluftzustand sich einstellt, besteht die Möglichkeit, über Rückluftsammler 19 und Rückluftregelventile 20, 21, 22 einen Teilstrom den Klimakammern 10, 11, 12 zuzuführen. Der Überschußluftstrom wird über das Absperrventil 51 an die Umgebung ausgeblasen. Der Gasstrahlventilator 18 wird über den Treibstrahlregler 18a derart einreguliert, daß ein minimaler Überdruck in den Klimakammern 10, 11, 12, herrscht. Dies ist erforderlich, um das Ansaugen von Umgebungsluft zu verhindern. Der Vorverdampfer 5 hat zwei Aufgaben zu erfüllen, einmal die optimale Nutzung der vorhandenen Kälteenergie aus der flüssigen Behandlungsatmosphäre und zum andern die Taupunktserniedrigung der bei 17 in den als Wärmetauscher ausgebildeten Vorverdampfer 5 ein strömenden Abluft aus den Klimakammern 10, 11, 12, um eine Rückluftführung zu den Klimakammern 10, 11, 12 zu ermöglichen.

Zum Mischen der Behandlungsphase in der Gasphase ist es theoretisch möglich, Sauerstoff und Stickstoff vor Vermischung zur Behandlungsatmosphäre aus den Lagertanks 1, 2 zuvor zu verdampfen. Dies geschied derart, daß die Absperrventile 63, 64 geöffnet werden und gleichzeitig die Absperrventile 59, 60 geschlossen sind. Der Verdampfer 65 wird derart über den Kreislaufventilator 66, das Heizregister 67 sowie die Temperaturregler 68, 69, eingeregelt, daß gerade beide Gase zu 100 % verdampft sind. Dies ist der Fall, wenn der Sauerstoff auf eine Temperatur in Abhängigkeit des herrschenden Druckes von ca. -180°C und der Stickstoff von ca. -192°C besitzt, was mittels der Temperaturregler 68, 69 geregelt wird. Das Absperrventil 55a muß dabei geschlossen sein. Danach besteht die Möglichkeit, den tiefkalten Gemischstrom über den Vorverdampfer 5 bei geöffnetem Absperrventil 53 und geschlossenem Absperrventil 78 zu leiten oder aber direkt zum Sammler 6 zu führen. Hierbei muß das Absperrventil 53 geschlossen und das Absperrventil 78 geöffnet sein. Die gesamte Regelung der Anlage 80 erfolgt danach wie oben beschrieben.

Es besteht auch die Möglichkeit, ohne Bevorratung im Lagertank 3 die gemischte Behandlungsatmosphäre direkt über den Vorverdampfer 5 in den Sammler 6 zu leiten. Hierbei müssen die Absperrventile 54, 55a, 78 geschlossen sein. In diesem Fall erfolgt die Regelung der Anlage 80 ebenfalls wie oben beschrieben.

Eine wichtige Voraussetzung für eine erfolgreiche Therapie ist die Vorbereitung der als Klimakammern ausgebildeten Behandlungskabinen sowie der Patienten. Die Klimakammern 10, 11, 12 werden vor Temperierung mit dem verdampften Gasgemisch bei Normaltemperatur geflutet. Die hierbei noch vorhandene Restfeuchtigkeit wird dadurch ausgetrieben und es treten beim Kaltfahren der Klimakabine 10, 11, 12 keine Vereisungen mehr auf. Um einen so geringen Feuchtigkeitseintrag in die Klimakammer 10, 11, 12 zu erhalten wie nur irgend möglich, ist es erforderlich, daß der Patient vor Mund und Nase einen Filter hat, der die ausgeatmete Atmosphäre von der Feuchtigkeit befreit. Dies kann z. B. durch ein Filter mit einem geeigneten Adsorbens erfolgen.

Der Volumenstrom des kalten trockenen Gasgemisches kann so bemessen werden, daß ein etwaiger Feuchtigkeitseintritt in der Klimakammer 10, 11, 12 mit der Abluft ausgetragen wird. Dieses kann durch eine Taupunktsregelung erfolgen, die einen Injektor ansteuert, der die abzuführende Abluftmenge einreguliert. Zweckmäßig ist es, alle mit tiefen Temperaturen beaufschlagten Anlagenkomponenten vakuumisoliert auszubilden. Dies ermöglicht die Optimierung der Betriebskosten und verhindert eine Kondensatbildung an den Oberflächen der Anlage. Vorteilhaft ist es, in den Klimakammern 10, 11, 12 mehrere Temperaturzonen auszubilden, die durch geeignete Schleusen voneinander getrennt sind. Hierdurch wird der Therapieablauf optimiert. Die Behandlungsatmosphäre kann während der Therapie z. B. durch Sauerstoffkonzentrationsveränderungen variiert werden. Zur Temperierung des Gasgemisches kann ein Verdampfer verwendet werden, der als Wärmeträger einen beheizten Kreislauf besitzt, der gegenüber der Normalatmosphäre hermetisch abgeschlossen ist. Hierdurch wird ein Vereisen der Verdampferanlage und somit eine Veränderung der Verdampferleistung verhindert. Der Wärmeträger kann ein nicht zu kondensierendes Gas wie z. B. Inertgas oder Edelgas sein. Voraussetzung hierbei ist, daß dieses Wärmeträgergas bei den zu erwartenden tiefen Temperaturen weder kondensiert noch kristallisiert. Ferner ist es möglich, als Wärmeträger eine nicht zu kristallisierende Flüssigkeit zu verwenden. Zwischen der Kabineninnenwand und der Kabinenaußenwand einer jeden Klimakammer 10, 11, 12 kann ein Ringspalt ausgebildet sein, der mit einem sehr trockenem Gas durchspült wird, das einen möglichst tiefen Taupunkt aufweist. Hierdurch wird bei in die Klimakammern 10, 11, 12 integrierten Fenstern deren Vereisung verhindert, so daß die Überwachung der Patienten nicht beeinträchtigt wird. Vor dem Eintritt in die Klimakammern 10, 11, 12 kann die Behandlungsatmosphäre ferner ionisiert werden. Es ist auch möglich, die Ionisierung der Behandlungsatmosphäre in den Klimakammern 10, 11, 12 selbst vorzunehmen.

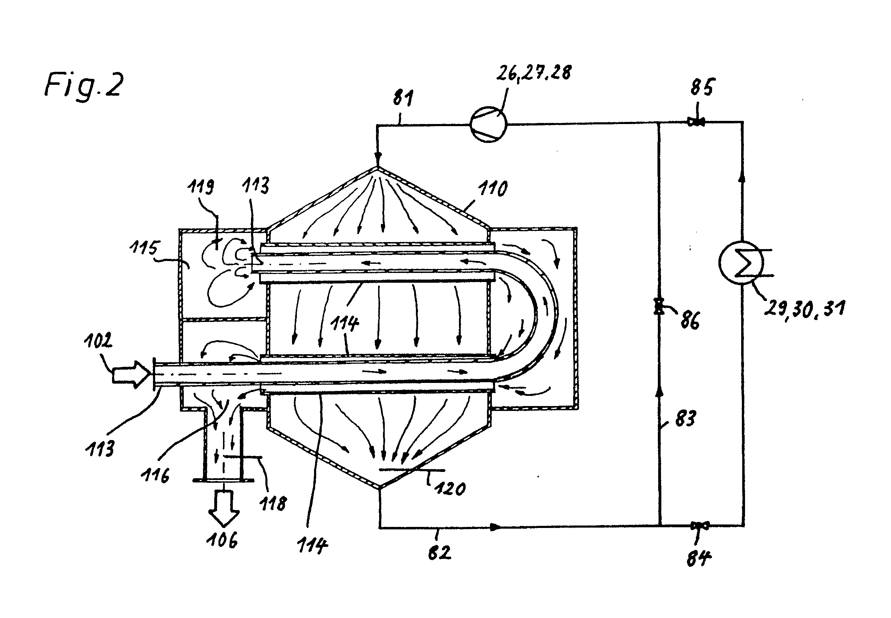

Um Edelgase oder Gasgemische zu verflüssigen, müssen sehr exakte Kondensationstemperaturen erzeugt werden. In den meisten Fällen ist die Temperatur des Behandlungsmediums 102 unter der Festpunkttemperatur der Edelgase oder aber der Tripelpunkt liegt ungünstig zur Temperatur des Behandlungsmediums 102. Dieses kann zu einer ungewollten Verfestigung der Edelgase und somit zu einem Vereisen des für die Temperierung des Behandlungsmedium 102 vorgesehenen Wärmetauscher führen. In Fig. 2 ist ein Wärmetauscher 101 schematisch dargetellt, der zur Erzielung einer exakten Temperatur des Behandlungsmediums 102 dient. Dieser Wärmetauscher 101 ist für alle Gase geeignet, die von einem tieferen Temperaturniveau auf ein höheres Temperaturniveau gebracht werden müssen. Hierbei ist es ohne Belang, ob das eintretene Medium flüssig oder gasförmig ist. Zweck der relativ aufwendigen Konstruktion ist die möglichst wirkungsvolle Ausnutzung der Wärmeübergangsparameter. Der Wärmetauscher 101 weist in einem Gehäuse 110 mindestens ein als Wärmetauscherrohr 111 dienendes gebogenes Doppelrohr 112 auf, das aus einem mit dem Behandlungsmediumeintrittsstutzen 105 verbundenen Kernrohr 113 und einem mit dem Behandlungsmediumaustrittsstutzen 106 verbundenen Mantelrohr 114 besteht. Das Kernrohr 113 ist mit dem Mantelrohr 114 mittels einer Umlenkkammer 115 verbunden. Der Behandlungsmediumaustrittsstutzen 106 ist mit dem Mantelrohr 114 mittels der Umlenkkammer 116 verbunden.

Das Behandlungsmedium 102 tritt in das Kernrohr 113 eines Mantelrohrs 114 ein und durchströmt das Kernrohr 113. Auf dem Weg zum Behandlungsmediumaustrittsstutzen 106 der Umlenkkammer 116 wird über das Mantelrohr 114 Wärme nur im geringen Maße aufgenommen, da die Rückströmung des Behandlungsmediums durch das Mantelrohr 114 gegenüber dem Heizmediumkreislauf 81, 82 als Isolation wirkt. Dies ist für das Regelverhalten in begrenzten Temperaturbereichen von Vorteil, ohne dabei den Wirkungsgrad des Wärmeübergangs zu beeinflussen Das im Mantelrohr 114 im Gegenstrom zum Kernrohr 173 befindliche gasförmige Behandlungsmedium wird durch den im Kreuzstrom befindlichen Heizmediumkreislauf 81, 82 weiter erwärmt. Gleichzeitig wird aber ein Teil dieser Wärme auf das im Kernrohr 113 befindliche gasförmige Behandlungsmedium abgegeben. Da der Austritt des Mantelrohrs 114 in der Umlenkkammer 116 im Eintrittsbereich des Behandlungsmediums 102 in das Kernrohr 113 angeordnet ist, besteht die Möglichkeit einer minimalen Rückkühlung des Behandlungsmediums am Behandlungsmediumaustrittstutzen 106, da die tiefste Temperatur im System am Eintritt des Behandlungsmediums 102 vorhanden ist. Dieser Rückkühleffekt verhindert eine Überhitzung der Behandlungsatmosphäre und erleichtert den Regelaufwand. Die für die Regelung erforderlichen Temperaturfühler 118, 119, 120 sind so angeordnet, daß Temperaturschwankungen schnell angezeigt werden und dementsprechende Regelvorgänge ausgelöst werden können. Der geschlossene Heizkreislauf 81, 82 enthält ein Gebläse 26, 27, 28 (Fig. 1) und ein Heizregister 29, 30, 31 (Fig. 1) und darüberhinaus eine Bypaßschaltung 83 mit den erforderlichen Schließorganen 84, 85, 86 zur raschen Temperatursenkung bei geänderten Therapiebedingungen.

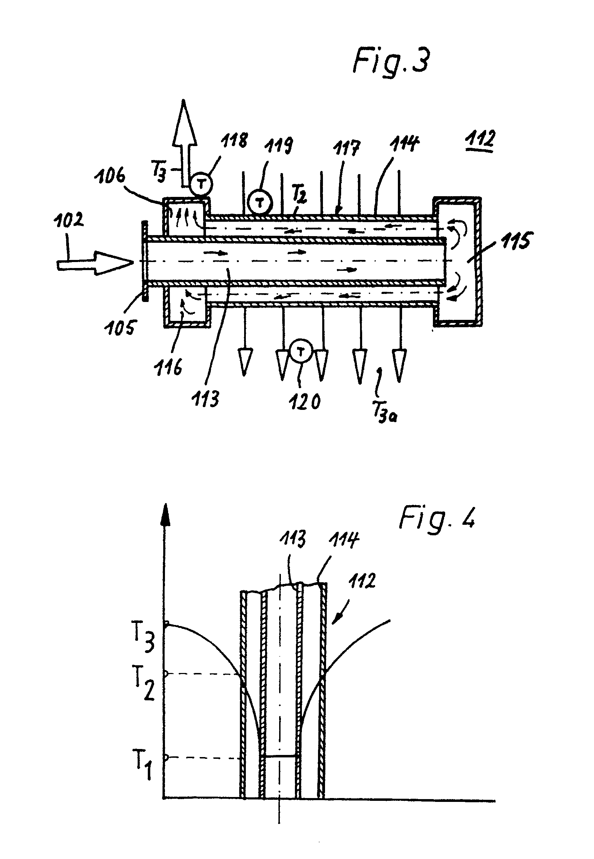

Bei dem Wärmetauscher 101 kann mit Hilfe des Doppelrohrs 112 eine exakte Wandtemperatur $T_2$ an der Außenfläche 117 des Mantelrohrs 114 eingeregelt werden (Fig. 3 und 4). Diese Wandtemperatur $T_2$ entspricht der Temperatur des Behandlungsmediums 102 in der gasförmigen Phase und liegt ca. 1° bis 2° C unter der kritischen Verfestigungstemperatur. Damit werden unkontrollierte Kristallbildungen und daraus folgende Veränderungen der Wärmedurchgangskoeffizienten verhindert. Ein optimaler Wärmeübergang ist somit gewährleistet. Darüberhinaus ist es aber auch möglich, eine Kristallisation bewußt herbei zu führen, indem die Wandtemperatur $T_2$ unter den Festpunkt der Edelgase oder Gasgemische eingestellt wird. Je nach Kapazität des Wärmetauschers (Kg Flüssigkeit/h) können verschiedene Wärmetauscherausführungen gewählt werden, die eine exakte Temperatur erreichen können. Für kleinere Leistungen eignet sich eine Spiralrohrausführung, für mittelgroße Leistungen eine Plattentauscherausführung und für große Leistungen eine Rohrbündelausführung. Der Ausnutzungsgrad und der Behandlungsmediumverbrauch ist jedoch höher und die Variationsmöglichkeiten sind nicht so vielfältig.

Für bewußte Kristallisationen können ergänzend konstruktive Hilfsmittel verwendet werden. Bei einer doppelten Aggregatausführung im wechselnden Betrieb kann der Vereisungsgrad über den Druckverlust ermittelt werden. Zum Reinigen vereister Wärmetauscherflächen können Kratzer oder Schaber dienen.

Zur optimalen Regelung der Temperatur im Wärmetauscher müssen die Temperaturen der den Wärmetauscher durchströmenden Mittel aufeinander abgestimmt werden, und zwar die Temperatur $T_2$ zur Grenztemperaturbestimmung und die Temperatur $T_3$ zur Regulierung des Behandlungsmediumeintritts, wobei bei Erreichung der Solltemperatur $T_2$ die Behandlungsmediumzufuhr unterbrochen wird. Es kann auch die Temperatur $T_2$ zur Grenztemperaturbestimmung und die Temperatur $T_{3a}$

zur Regulierung des Behandlungsmediumeintritts verwendet werden. Wie bereits beschrieben, ist die Temperatur $T_2$ der Temperatur $T_{3a}$ übergeordnet.

Das Verfahren und die Anlage wie oben beschrieben zeichnet sich durch folgende Vorteile aus:

- Betriebsbereitschaft der Behandlungskabine innerhalb von 30 Minuten,
- Taupunkt der Behandlungsatmosphäre -175° C,
- der Einsatz von Kältemaschinen entfällt,
- Reduzierung der Betriebskosten,
- Reduzierung des Wartungsaufwandes,
- Variationsmöglichkeiten der Sauerstoffkonzentration der Behandlungsatmosphäre,
- keine Nebelbildung in der Behandlungskabine,
- es können Behandlungstemperaturen bis zu -175° C erreicht werden.

Bevor die Behandlungskabine auf Tiefentemperatur gebracht werden kann, sind folgende Schritte durchzuführen:

- Bereitstellung von flüssigem Sauerstoff und Stickstoff in ausreichender Menge und Qualität. Beide Gase werden in superisolierten Vakuumbehältern gelagert. Beide Lagertanks 1, 2 müssen mit einer Konstantdruckregelung und Niveauüberwachung ausgerüstet sein, um einen sicheren und störungsfreien Verfahrensablauf zu gewährleisten.
- Alle Analysengeräte müssen auf ihre Funktionsfähigkeit hin überprüft und falls erforderlich eingeeicht werden. Dies gilt besonders für die Sauerstoffkonzentrationsanalyse.
- Mischen beider Gase in einem Verhältnis derart, daß die gewünschte Sauerstoffkonzentration vorliegt. Auch dieses Gemisch muß überprüft und analysiert werden. Das Gemisch liegt noch in flüssiger Form vor und wird in den dritten Lagertank 3 gefüllt. Auch dieser Lagertank 3 verfügt über eine Konstantdruckregelung und Niveauüberwachung .
- Das flüssige Sauerstoff/Stickstoff-Gemisch wird über einen Verdampfer geleitet und auf eine Temperatur gebracht, die nicht unter der normalen Umgebungstemperatur der Behandlungskabine liegt. Mit dieser sehr trockenen Behandlungsatmosphäre wird die Behandlungskabine geflutet. Der Flutvorgang wird solange durchgeführt, bis der Taupunkt der Kabinenatmosphäre dem gewünschten Wert entspricht. Dies ist erforderlich, um ein Vereisen und Nebelbildung in der Behandlungskabine zu vermeiden.

Nach diesen Maßnahmen ist die Kabine für das Kaltfahren auf Behandlungstemperatur vorbereitet, für das folgende Verfahrensschritte erforderlich sind:

- Überprüfung der Behandlungsatmosphäre auf Sauerstoffkonzentration, Taupunkt und Füllniveau Lagertank.
- Verdampfen der Behandlungsatmosphäre auf die gewünschte Therapietemperatur.
- Einregulierung der Kabinenabluft derart, daß in der Behandlungskabine ein minimaler Überdruck zum Umgebungsdruck herrscht. Dies ist erforderlich, um eine Ansaugung feuchter Luft aus der Umgebung zu verhindern.
- Die Behandlungskabine ist nach Erreichung der Therapietemperatur einsatzbereit.

Die durchströmende Menge an Behandlungsatmosphäre ist so ausgelegt, daß ein mindestens 3-facher Atmosphärenwechsel pro Stunde erreicht wird. Kälteaggregate zur Erzeugung der Tieftemperatur werden nicht benötigt. Dies gilt auch für die Taupunktserniedrigung der Behandlungsatmosphäre. Für einen störungsfreien Ablauf der Therapie ist es erforderlich, alle mit der Behandlungsatmosphäre zusammenhängenden Phänomene permanent zu überwachen und gegebenenfalls automatisch regelnd einzugreifen. Folgende Schritte sind dafür erforderlich:

- Überwachung der Sauerstoffkonzentration permanent und falls erforderlich Nachregulierung über Steuerventile zur Zugabe von Sauerstoff oder Reduzierung von Stickstoff.
- Überwachung des $CO_2$-Gehaltes der Behandlungsatmosphäre und falls erforderlich Erhöhung des Volumenstromes der Behandlungsatmosphäre und gleichzeitige Erhöhung der Abluftmenge unter Aufrechterhaltung des Überdrucks in der Behandlungskabine.
- Überwachung des Drucktaupunktes und wenn erforderlich Erhöhung des Volumenstromes der Behandlungsatmosphäre wie oben .
- Überwachung der Therapietemperatur und falls erforderlich Regulierung über die Verdampfereinheit.
- Überwachung der Rückluftqualität hinsichtlich Sauerstoffkonzentration , $CO_2$-Konzentration, Taupunkt und Temperatur. Falls erforderlich kann die Rückluftmenge entsprechend ihrer Qualität eingeregelt werden.

Die Überwachung der Behandlungsatmosphäre ist für den Erfolg der Therapie und Sicherheit der Patienten von großer Bedeutung. Außerdem wird mit diesen Regelmöglichkeiten ein Vereisen und Nebelbildung in der Kabine verhindert. Die Flexibilität der Flüssiggasversorgung läßt einen breiten Leistungsbereich zu. Dies bedeutet, daß eine Erweiterung der Anlagenkapazität ohne weiteres möglich ist. Alle vor Eintritt in die Behandlungskabine angeordneten Aggregate können nicht vereisen und behalten ihre volle Leistung auch beim Langzeitbetrieb bei. Eine individuelle Einregulierung der Sauerstoffkonzentration von 20,9 Vol. % bis zur reinen Sauerstoffatmosphäre ist ohne zusätzlichen Aufwand möglich. Es können beliebig viele Temperaturzonen angesteuert werden ohne eine Veränderung in der Flüssigversorgung vorzusehen. Die Nutzung der gesamten zur Verfügung stehenden Kälteenergie aus der Flüssigbevorratung ist gegeben. Es besteht die Möglichkeit zur Behandlungsatmosphäre Wirksubstanzen einzugeben, die den Therapieerfolg verbessern können. Die Behandlungsatmosphäre unterliegt ständigen Überwachungen durch Massenspektralanalysen und kann jederzeit qualitativ und quantitativ beurteilt werden.

Besonders bei kleineren Anlagen zur Erzeugung einer Behandlungsatmosphäre ist es auch möglich, statt einer getrennten Lagerung von $O_2$ und $N_2$ in den Lagertanks 1, 2 mit anschließender Mischung der Gase einen Luftverflüssiger vorzusehen. In diesem Fall kann der verflüssigten Luft $O_2$ beigemischt werden, damit das Mischungsverhältnis der Behandlungsatmosphäre optimiert wird.

**Patentansprüche**

1. Verfahren zur Erzeugung einer Behandlungsatmosphäre für die Ganzkörperbehandlung rheumatisch erkrankter Personen durch Tieftemperaturtherapie, bei dem in einem etwa der Zusammensetzung von Normalluft entsprechendem Mischungsverhältnis Stickstoff und Sauerstoff mit tiefer Temperatur in eine Behandlungskabine eingeleitet werden, dadurch gekennzeichnet,

   daß entweder flüssiger Sauerstoff und flüssiger Stickstoff, die in flüssiger Phase gemischt oder auf eine von der gewünschten Behandlungstemperatur abhängige Temperatur verdampft und dann gemischt werden, oder in einer Luftverflüssigungsanlage verflüssigte Luft, der gegebenenfalls Sauerstoff zugemischt wird,

   entweder über einen Vorverdampfer, über eine Nachheizeinrichtung, über einen Vorverdampfer und eine Nachheizeinrichtung oder - falls schon verdampft - direkt volumenstromgeregelt in die Behandlungskabine eingeleitet werden.

2. Verfahren nach Anspruch 1, dadurch gekennzeichnet, daß der Taupunkt durch Steuerung der abgesaugten Abluftmenge aus der Behandlungskabine geregelt wird.

3. Verfahren nach Anspruch 1 oder 2,dadurch gekennzeichnet, daß die Abluft teilweise in die Behandlungskabine rückgeführt wird.

4. Verfahren nach Anspruch 3, dadurch gekennzeichnet, daß die Abluft im Wärmeaustausch mit dem zugeführten Sauerstoff-/Stickstoffgemisch rückgeführt wird.

5. Verfahren nach einem der Ansprüche 1 bis 4, dadurch gekennzeichnet, daß die Sauerstoffkonzentration der Behandlungsatmosphäre während der Therapie durch Volumenstromregelung geändert wird.

6. Verfahren nach einem der Ansprüche 1 bis 5, dadurch gekennzeichnet, daß die in die Behandlungskabine eingebrachte Behandlungsatmosphäre auf ihre Kohlendioxidkonzentration überwacht wird.

7. Verfahren nach Anspruch 1, dadurch gekennzeichnet, daß zur Mischung in der Gasphase der flüssige Sauerstoff und der flüssige Stickstoff vor dem Mischen verdampft werden.

8. Verfahren nach Anspruch 7, dadurch gekennzeichnet, daß der Wärmeträger zum Verdampfen in einer inerten, beheizten, gegenüber der Normalatmosphäre hermetisch abgeschlossenen Atmosphäre gehalten ist.

9. Verfahren nach Anspruch 8, dadurch gekennzeichnet, daß als Wärmeträger ein nicht zu kondensierendes Gas oder eine nicht zu kristallisierende Flüssigkeit verwendet wird.

10. Verfahren nach einem der Ansprüche 1 bis 9, dadurch gekennzeichnet, daß die Behandlungsatmosphäre vor Eintritt in die Behandlungskabine ionisiert wird.

11. Verfahren nach einem der Ansprüche 1 bis 9, dadurch gekennzeichnet, daß die Behandlungsatmosphäre in der Behandlungskabine ionisiert wird.

12. Verfahren nach einem der Ansprüche 1 bis 11, dadurch gekennzeichnet, daß vor dem Herstellen der tiefgekühlten Behandlungsatmosphäre in der Behandlungskammer diese zunächst mit einem Stickstoff-/Sauerstoffgemisch bei Normaltemperatur gespült wird, bis eine vorgegebene Taupunkttemperatur erreicht worden ist.

13. Anlage zur Durchführung des Verfahrens nach einem der Ansprüche 1 bis 12, mit mindestens einer Behandlungskabine und einer an die Behandlungskabine angeschlossenen Mischvorrichtung zur Herstellung einer aus tiefgekühltem Sauerstoff und Stickstoff bestehenden Behandlungsatmosphäre in einer der natürlichen Atmosphäre entsprechenden Konzentration, gekennzeichnet durch mindestens zwei Lagertanks (1, 2) für flüssigen Stickstoff und flüssigen Sauerstoff, die jeweils mittels einer Leitung (81, 82) mit Volumenstromreglern (76, 77) mit einer Mischkammer (4) verbunden sind, deren Ausgang mit mindestens einem Lagertank (3) für die Behandlungsatmosphäre verbunden ist, der seinerseits über eine geregelte Nachheizeinrichtung (7, 8, 9) mit der Behandlungskabine (10, 11, 12) verbunden ist.

14. Anlage nach Anspruch 13, dadurch gekennzeichnet, daß zwischen der Kabinenaußenwand und der Kabineninnenwand ein mit einem sehr trockenen Tieftemperaturgas durchspülbarer Raum vorgesehen ist.

15. Anlage nach Anspruch 14, dadurch gekennzeichnet, daß alle mit tiefen Temperaturen belasteten Anlagekomponenten vakuumisoliert sind.

16. Anlage nach Anspruch 13, dadurch gekennzeichnet, daß zwischen den Lagertanks (1, 2) und der Mischkammer (4) ein Verdampfer (65) mit einem gegenüber Normalatmosphäre hermetisch abgeschlossenen, inerten beheizten Kreislauf für den Wärmeträger angeordnet ist.

17. Anlage nach Anspruch 16, dadurch gekennzeichnet, daß am Ausgang der Mischkammer (4) ein Meßgerät (61, 62) für die Ermittlung der Sauerstoffkonzentration des Taupunktes angeordnet ist.

18. Anlage nach einem der Ansprüche 13 bis 17, dadurch gekennzeichnet, daß eine Temperaturregelkammer (7, 8, 9) als Nachheizeinrichtung für einen Sammler (6) ausgebildet ist, deren Ausgang mit einem Rücklaufsammler (19) verbunden ist.

19. Anlage nach einem der Ansprüche 13 bis 18, dadurch gekennzeichnet, daß der Nachheizvorrichtung (7, 8, 9) ein Vorverdampfer (5) vorgeschaltet ist.

20. Anlage nach Anspruch 19, dadurch gekennzeichnet, daß der Absauganschluß der Behandlungskabine (10, 11, 12) mit dem Vorverdampfer (5) verbunden ist.

21. Anlage nach einem der Ansprüche 13 bis 20, dadurch gekennzeichnet, daß der Nachheizvorrichtung (7, 8, 9) ein Volumenstromregler (23, 24, 25) vorgeschaltet ist.

22. Anlage nach einem der Ansprüche 13 bis 21, dadurch gekennzeichnet, daß vor dem Eintritt der Behandlungsatmosphäre in die Behandlungskammer (10, 11, 12) und am Ausgang des Vorverdampfers (5) jeweils ein Meßgerät für die Ermittlung der Sauerstoffkonzentration (47, 32, 36, 40) der Taupunkttemperatur (48, 33, 37, 41) und für die Analyse des $CO_2$-Gehalts (49, 34, 38, 42) angeordnet ist.

23. Anlage nach Anspruch 22, dadurch gekennzeichnet,daß an dem Vorverdampfer (5) ein Meßgerät (50) für die Ermittlung der Temperatur des Gasgemisches angeordnet ist.

24. Anlage nach Anspruch 22, dadurch gekennzeichnet, daß vor dem Eingang der Behandlungskammer (10, 11, 12) ein Temperaturmeßgeber (35, 39, 43) angeordnet ist.

**Claims**

1. A method for generating a treating atmosphere for the entire body therapy of rheumatically diseased persons by a deep temperature therapy, wherein nitrogen and oxygen of a deep temperature in a proportion substantially corresponding to the normal air are introduced to a treating cabin,
characterized in that
either a mixture of liquid oxygen and liquid nitrogen mixed in the liquid state or evaporated to a temperature in response to the desired treating temperature and then mixed, or air liquidized in an air liquidifying apparatus which air may be mixed with oxygen,
is supplied to the treating cabin either through a pre-evaporator, an after-heater, a pre-evaporator and an after-heater or - when evaporated - directly by controlling the volume flow.

2. The method of claim 1, characterized in that the dew point is regulated by controlling the amount of exhaust air sucked out of the treating cabin.

3. The method of claim 1 or 2, characterized in that the exhaust air is partly returned to the treating cabin.

4. The method of claim 3, characterized in that the exhaust air is returned in a heat exchange relation with the oxygen-nitrogen mixture supplied.

5. The method of one of claims 1 to 4, characterized in that the oxygen concentration of the treating atmosphere is varied by volume flow control during the therapy.

6. The method of one of claims 1 t 5, characterized in that the treating atmosphere supplied to the treating cabin is controlled with respect to its $CO_2$ concentration.

7. The method according to claim 1, characterized in that for the mixing in the gas phase the liquid oxygen and the liquid nitrogen are evaporated before being supplied to a mixing chamber.

8. The method according to claim 7, characterized in that the heat carrier for evaporation is held in an inert, heated atmosphere hermetically closed with respect to the normal atmosphere.

9. The method of claim 8, characterized in that a non-condensible gas or a non-crystallizable liquid is used as heat carrier.

10. The method according to one of the claims 1 to 9, characterized in that the treating atmosphere is ionized before its entrance to the treating cabin.

11. The method of one of claims 1 to 9, characterized in that the treating atmosphere is ionized within the treating cabin.

12. The method of one of claims 1 to 11, characterized in that before creating the deep cooled treating atmosphere in the treating cabin, the cabin is first rinsed with a nitrogen-oxygen mixture at normal temperature until a predetermined dew temperature is obtained.

13. An apparatus for carrying out the method of one of claims 1 to 12, comprising at least a treating cabin and a mixing device connected to the treating cabin for generating a treating atmosphere comprising deep cooled oxygen and nitrogen in a concentration corresponding to the natural atmosphere, characterized by at least two storage tanks (1,2) for liquid nitrogen and liquid oxygen, the storage tanks being connected to a mixing chamber (4) each through a conduit (81,82) including volume flow regulators (76,77), the outlet of the mixing chamber being connected to at least a storage tank (3) for the treating atmosphere which storage tank is connected to the treating cabin (10,11,12) through a controlled post-heating means (7,8,9).

14. The apparatus of claim 13, characterized in that a space which can be rinsed with a very dry deep temperature gas is provided between the cabin outer wall and the cabin inner wall.

15. The apparatus of claim 14, characterized in that all system components exposed to deep temperatures are vacuum insulated.

16. The apparatus of claim 13, characterized in that an evaporator (65) is provided between the storage tanks and the mixing chamber (4) which evaporator includes an inert heated circuit for the heat carrier and is hermetically closed off with respect to the normal atmosphere.

17. The apparatus of claim 16, characterized in that a measuring device (61,62) is provided at the outlet of the mixing chamber (4) for determining the oxygen concentration of the due point.

18. The apparatus of one of claims 13 to 17, characterized in that a temperature control chamber (7,8,9) is formed as post-heating means for a collector (6), the outlet thereof being connected to a back-flow collector.

19. The apparatus of one of claims 13 to 18, characterized in that a pre-evaporator (5) is provided upstream of the post-heating means (7,8,9).

20. The apparatus of claim 19, characterized in that the exhaust port of the treating cabin (10,11,12) is connected to the pre-evaporator (5).

21. The apparatus of one of claims 13 to 20, characterized in that a volume flow regulator (23,24,25) is provided upstream of the post-heating means (7,8,9).

22. The apparatus of one of claims 13 to 21, characterized in that a measuring device each for determining the oxygen concentration (47,32,36,40) for determining the dew point temperature (48,33,37,41) and for the analysis contents (49,34,38,42) is provided upstream of the entrance of the treating atmosphere to the treating cabin (10,11,12) and at the outlet of the pre-evaporator (5).

23. The apparatus of claim 22, characterized in that the pre-evaporator (5) is provided with a measuring device (50) for determining the temperature of the gas mixture.

24. The apparatus of claim 22, characterized in that a temperature sensor (35,39,43) is provided upstream of the entrance to the treating cabin (10,11,12).

## Revendications

1. Procédé d'obtention d'une atmosphère de traitement de personnes souffrant de rhumatismes par une thérapie à basse température, selon lequel on envoie, dans une cabine de traitement, un mélange d'azote et d'oxygène à basse température ayant à peu près la composition de l'air normal,
   caractérisé en ce que
   - soit de l'oxygène liquide et de l'azote liquide, qui ont été mélangés à l'état liquide ou qui ont été vaporisés à une température qui dépend de la température de traitement et mélangés ensuite,
   - soit de l'air qui a été liquéfié dans une installation de liquéfaction de l'air et auquel on a éventuellement ajouté de l'oxygène,
   sont envoyés dans la cabine, avec un débit en volume régulé :
   - soit
     à travers un prévaporisateur,
     à travers un dispositif de post-chauffage
     à travers un prévaporisateur et un dispositif de post-chauffage,
   - soit directement, au cas où ils sont déjà vaporisés,

2. Procédé selon la revendication 1, caractérisé en ce que le point de rosée est régulé par commande du débit de sortie de l'air aspiré de la cabine de traitement.

3. Procédé selon la revendication 1 ou 2, caractérisé en ce que l'air sortant de la cabine de traitement est partiellement recyclé.

4. Revendication selon la revendication 3, caractérisée en ce que l'air sortant est recyclé dans un échangeur de chaleur avec l'arrivée de mélange d'oxygène et d'azote.

5. Procédé selon l'une des revendications 1 à 4, caractérisé en ce que l'on fait varier la concentration en oxygène de l'atmosphère de traitement pendant la thérapie à l'aide d'une régulation de débit en volume.

6. Procédé selon l'une des revendications 1 à 5, caractérisé en ce que l'on contrôle l'atmosphère indroduite dans la cabine de traitement en ce qui concerne sa concentration en bioxyde de carbonne.

7. Procédé selon la revendication 1, caractérisé en ce que l'oxygène liquide et l'azote liquide sont vaporisés avant d'être mélangés, et sont mélangés en face gazeuse.

8. Procédé selon la revendication 7, caractérisé en ce que le moyen caloporteur pour la vaporisation est maintenu dans une atmosphère inerte, chauffée, et complètement isolée par rapport à l'atmosphère normale.

9. Procédé selon la revendication 8, caractérisé en ce que l'on utilise comme moyen caloporteur un gaz non condensable ou un liquide non cristallisable.

10. Procédé selon l'une des revendications 1 à 9, caractérisé en ce que l'on ionise l'atmosphère de traitement avant son entrée dans la cabine de traitement.

11. Procédé selon l'une des revendications 1 à 9, caractérisé en ce que l'on ionise l'atmosphère de traitement dans la cabine de traitement.

12. Procédé selon l'une des revendications 1 à 11, caractérisé en ce que, avant d'établir l'atmosphère de traitement à basse température dans la chambre de traitement, on envoie dans celle-ci un mélange d'azote et d'oxygène à température normale, jusqu'à ce que l'on ait atteint une température de point de rosée fixée à l'avance.

13. Installation pour la mise en oeuvre du procédé selon l'une des revendications de 1 à 12, comprenant au moins une cabine de traitement et une installation de mélange, reliée à la cabine de traitement, pour l'obtention d'une atmosphère de traitement composée d'oxygène et d'azote à basse température à une concentration correspondant à celle de l'atmosphère naturelle, caractérisée par au moins deux réservoirs de stockage (1, 2) pour l'azote liquide et l'oxygène liquide, qui sont reliés chacun au moyen d'une conduite (81, 82) pourvue de dispositifs de réglage de débit volumétriques (76, 77), à une chambre de mélange (4), dont la sortie est reliée à au moins un réservoir de stockage (3) pour l'atmosphère de traitement, lequel de son côté est relié à la cabine de traitement (10, 11, 12) à travers un dispositif régulé de post-chauffage (7, 8, 9).

14. Installation selon la revendication 13, caractérisée en ce qu'il est prévu, entre les parois extérieures et intérieures de la cabine, un espace qui peut être balayé par un gaz à basse température, très sec.

15. Installation selon la revendication 14 caractérisée en ce que tous les composants de l'installation qui sont à basse température sont isolés par le vide.

16. Installation selon la revendication 13, caractérisée en ce qu'un vaporisateur (65) pourvu d'un circuit fermé d'un produit caloporteur, chauffé, inerte, et hermétiquement isolé de l'atmosphère normale, est prévu entre les réservoirs de stockage (1, 2) et la chambre de mélange (4).

17. Installation selon la revendication 16, caractérisée en ce qu'un dispositif de mesure (61, 62) pour la détermination de la concentration en oxygène et/ou du point de rosée est disposé à la sortie de la chambre de mélange (4).

18. Installation selon l'une des revendications 13 à 17, caractérisée en ce qu'une chambre de régulation de température (7, 8, 9) est disposée comme dispositif de post-chauffage pour un collecteur (6), dont la sortie est reliée à un collecteur de recyclage (19).

19. Installation selon l'une des revendications 13 à 18, caractérisée en ce qu'un prévaporisateur (5) est disposé en amont du dispositif de post-chauffage (7, 8, 9).

20. Installation selon la revendication 19 caractérisée en ce que la sortie de la cabine de traitement (10, 11, 12) est reliée au prévaporisateur (5).

21. Installation selon l'une des revendications 13 à 20, caractérisée en ce qu'un régulateur de débit en volume (23, 24, 25) est disposé en amont du dispositif de post-chauffage (7, 8, 9).

22. Installation selon l'une des revendications 13 à 21, caractérisée en ce que des dispositifs de mesure pour la détermination de la concentration en oxygène (47, 32, 36, 40), de la température du point de rosée (48, 33, 37, 41), et l'analyse de la teneur en $CO_2$ (49, 34, 38, 42) sont disposés avant l'entrée de l'atmosphère de traitement dans la chambre de traitement (10, 11, 12), et à la sortie du prévaporisateur (5).

23. Installation selon la revendication 22, caractérisée en ce qu'un dispositif de mesure (50) pour la détermination de la température du mélange gazeux est associé au prévaporisateur (5).

24. Installation selon la revendication 22, caractérisée en ce qu'un dispositif de mesure de température (35, 39, 43) est disposé avant l'entrée de la chambre de traitement (10, 11, 12).

Fig.1

EP 0 226 107 B1

Fig.2

## Fig. 3

## Fig. 4